# EUROPÄISCHE PATENTSCHRIFT

(11) **EP 1 471 349 B1**
(45) Veröffentlichungstag und Bekanntmachung des Hinweises auf die Patenterteilung: **28.12.2011**
(21) Anmeldenummer: 04006756.3
(22) Anmeldetag: 20.03.2004
(51) Int. Cl.: G01N 27/90, G01N 27/26

(54) **Potentiometrische Sensoreinrichtung für pH-Wertmessung**
Potentiometric sensor device for pH measurement
Capteur potentiométrique pour la mesure de pH

(30) Priorität: 22.04.2003 DE 10318115
(43) Veröffentlichungstag der Anmeldung: 27.10.2004
(73) Patentinhaber: ROBERT BOSCH GMBH, 70442 Stuttgart (DE)
(72) Erfinder: Scheying, Gerd, 70186 Stuttgart (DE); Lewis, Mary, 70839 Gerlingen (DE)

(56) Entgegenhaltungen:
- DE-A1- 4 430 662
- DE-A1- 10 062 044
- DE-A1- 19 612 680
- DE-C1- 19 544 690
- US-A- 5 141 868
- US-A1- 2003 047 450

## Beschreibung

### Stand der Technik

Die Erfindung betrifft eine potentiometrische Sensoreinrichtung gemäß der im Oberbegriff des Patentanspruches 1 näher definierten Art.

Aus der Praxis bekannte potentiometrische Sensoren beruhen auf dem Prinzip, daß zwischen mindestens zwei Elektroden eine elektrische Spannung gemessen werden kann, die sich mit der Änderung einer chemischen Spezieskonzentration ändert. Die zu messende Spannung wird durch einen elektrischen Potentialunterschied hervorgerufen, der aus einer Differenz des chemischen Gleichgewichtspotentials der einzelnen Elektroden mit deren Umgebung resultiert.

Derartige Sensoren werden beispielsweise als Lambdasonden oder als schwefelsaure elektrochemische Zelle zur Messung von Kohlenmonoxid verwendet.

Des Weiteren werden zur Messung von sauren Bestandteilen in Flüssigkeiten oder Gasen, d. h. zur Ermittlung einer Wasserstoffionen-Konzentration bzw. eines pH-Wertes, sogenannte Gaselektroden bzw. Standard-Wasserstoffelektroden in meist wäßrigen Medien verwendet. Ferner werden sogenannte Membranelektroden, wie eine pH-Glaselektrode oder auch andere Festkörper mit ionensensitiver Wirkung, zur Messung von pH-Werten in flüssigen Medien eingesetzt.

Aus der US 2003/047450 A1 ist eine potentiometrische Sensoreinrichtung zur pH-wert-Messung bekannt, deren Elektroden eine Interdigitalstruktur haben.

Eine weitere Sensoreinrichtung ist aus der DE 196 12 980 A1 bekannt, bei der Elektroden zur Erhöhung einer Sensitivität mit einer kationenselektiven Schicht beschichtet sind, die unter anderem mit Polyamidweichmachern ausführbar ist.

Nachteilig dabei ist jedoch, daß die bekannten und zur pH-Wert-Messung verwendeten Sensoreinrichtungen hohe Herstellkosten verursachen und zudem in Bezug auf mechanische und/oder chemische Belastungen nur eine geringe Widerstandsfähigkeit aufweisen.

Die potentiometrische Sensoreinrichtung zur pH-Wert-Messung mit den Merkmalen des Patentanspruches 1 hat demgegenüber den Vorteil, daß sie günstig herstellbar ist und zudem eine sehr robuste Ausführung einer Sensoreinrichtung zur pH-Wert-Messung darstellt.

Dies wird dadurch erreicht, daß die Elektroden der erfindungsgemäßen Sensoreinrichtung mittels Dickschichttechnik in einer Interdigitalstruktur auf dem Substrat aufgebracht sind, wodurch die Sensoreinrichtung in einer Massenfertigung herstellbar ist. Des Weiteren ist die Sensoreinrichtung aufgrund der Interdigitalstruktur ohne eine Referenzelektrode mit Glaskörper ausführbar, so daß die Sensoreinrichtung auch in Automobilen und industriellen Anlagen, wo die Sensoreinrichtung hohen mechanischen Belastungen ausgesetzt ist, problemlos einsetzbar ist.

Des Weiteren weist die erfindungsgemäße Sensoreinrichtung aufgrund der auf den Elektroden vorgesehenen wasserhaltigen Schicht, die aus Polyamid, vorzugsweise PA 4, PA 4.4, PA 6, PA 6.6 oder PA 12, Polyimid, Polyacrylat, Polyethylenglykol, Zellulose oder einem Zellulosederivat besteht, im Vergleich zu bekannten Sensoreinrichtungen eine verbesserte Sensitivität auf und ist auch gegenüber Motoröl mit einer ausreichenden Beständigkeit ausgeführt, womit sich die Eigenschaften der wasserhaltigen Schicht im Betrieb der Sensoreinrichtung nicht verändern.

Weitere Vorteile und vorteilhafte Ausgestaltungen des Gegenstandes nach der Erfindung sind der Beschreibung, der Zeichnung und den Patentansprüchen entnehmbar.

### Zeichnung

Ein Ausführungsbeispiel einer erfindungsgemäßen potentiometrischen Sensoreinrichtung zur pH-Wert-Messung ist in der Zeichnung schematisiert dargestellt und wird in der nachfolgenden Beschreibung näher erläutert. Es zeigen
Figur 1 eine Prinzipskizze einer als Ölzustandssensor ausgeführten potentiometrischen Sensoreinrichtung nach der Erfindung mit einer Auswerteschaltung, und
Figur 2 den in Figur 1 dargestellten Bereich X in vergrößerter Einzeldarstellung.

### Beschreibung des Ausführungsbeispiels

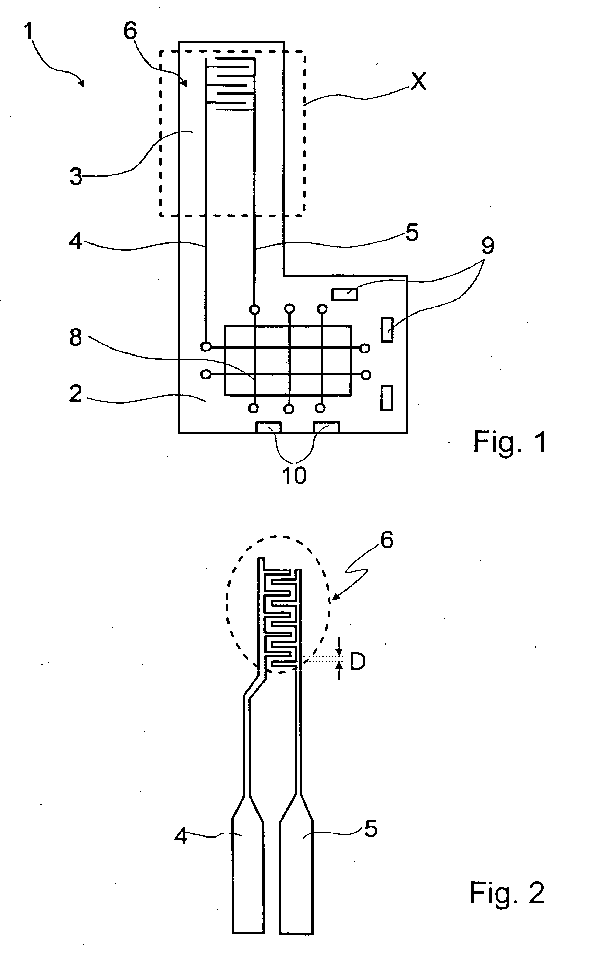

In Figur 1 ist eine Prinzipskizze einer potentiometrischen Sensoreinrichtung 1 dargestellt, mittels der ein pH-Wert in einem flüssigen Medium bestimmbar bzw. meßbar ist. Die potentiometrische Sensoreinrichtung 1 ist vorliegend als ein Ölzustandssensor mit einer Auswerteschaltung 2 ausgeführt, mit der ein Alterungsprozeß eines Motoröles einer Brennkraftmaschine eines Kraftfahrzeuges sensiert werden kann. Damit ist ein Zustand eines Motoröles permanent feststellbar, und ein Zeitpunkt eines erforderlichen Ölwechsels ist in Abhängigkeit eines tatsächlichen Zustandes des Motoröles ermittelbar und einem Fahrer anzeigbar.

Die Sensoreinrichtung 1 ist mit einem Substrat 3 ausgeführt, auf welchem zwei Elektroden 4, 5 und die Auswerteschaltung 2 angeordnet sind. Das Substrat 3 ist vorliegend als eine keramische Folie aus einem niedrigsinternden Glas-Keramiksubstrat, wie einer Low-Temperature-Cofiring-Ceramic (LTCC), gebildet, welche durch eine sehr geringere elektrische Leitfähigkeit und eine sehr gute mechanische Stabilität gekennzeichnet ist. Alternativ hierzu kann das Substrat auch aus Aluminiumoxid, Aluminiumnitrid, Siliziumdioxid oder einem Wafer aus Silizium, Siliziumnitrid oder dergleichen hergestellt sein.

Die beiden Elektroden 4 und 5 sind in Dickschichttechnologie mit einer interdigitalen Kammstruktur bzw. mit einer Interdigitalstruktur 6 auf dem Substrat 3 aufgebracht, wobei die Elektroden 4 und 5 vorliegend über ein Siebdruckverfahren in Form von Pasten aus Elektrodenmaterial aufgebracht sind. Die vorgenannten Elektrodenpasten sind aus dem jeweiligen Elektrodenmaterial in Pulverform und einem anorganischen oder organischen Vehikel gebildet, wobei das Elektrodenmaterialpulver bei der Pastenherstellung in dem Vehikel dispergiert wird.

Entsprechend einer Siebbeschaffenheit werden auf dem Substrat während des Siebdruckverfahrens Elektroden mit einer Schichthöhe zwischen 10 µm und 20 µm aufgebracht. Anschließend werden die pastösen Elektroden gemeinsam mit dem Substrat 3 in einem Brennprozeß gebrannt, wobei sich zwischen dem Substrat 3 und den Elektroden eine feste Verbindung ausbildet und das Vehikel der Pasten verdampft, was wiederum zu einer Reduzierung der Schichthöhen führt.

Alternativ hierzu kann es auch vorgesehen sein, daß die Elektroden mittels eines anderen aus der Dickschichttechnik bekannten Druckverfahrens, wie beispielsweise Schablonendruck oder Foliendruck, oder auch durch Inkjet-Technik auf dem Substrat aufgetragen werden.

Die zunächst in Pulverform vorliegenden Elektrodenmaterialien werden zur Verbesserung einer Haftung auf dem Substrat 3 jeweils getrennt mit einem anorganischen Pulver gemischt, wobei das anorganische Pulver vorliegend dem Substratmaterial entspricht. Das anorganische Material weist dabei Bestandteile auf, die während des Brennprozesses der Sensoreinrichtung aufschmelzen und mit dem Substrat einen Stoffschluß durch molekulare Kräfte zwischen den Molekülen des Substrats und des anorganischen Materials ausbilden.

In Abhängigkeit des jeweils vorliegenden Anwendungsfalles beträgt der Anteil des Substratmaterials zwischen 0,2 Massen-% und 20 Massen-% Pulver in der Elektrodenpaste, wobei mit Anteilen zwischen 10 Massen-% und 15 Massen-% an anorganischem Pulver ein besonders gutes Haften der Elektroden 4 und 5 auf dem Substrat 3 erreicht wird. Das Gemisch aus dem Elektrodenmaterialpulver und dem anorganischen Pulver wird vor dem Auftragen mit dem anorganischen oder organischen Vehikel vermischt bzw. in dem Vehikel dispergiert, wobei das Verhältnis zwischen einer Vehikelmasse und einer Pulvergemischmasse in Abhängigkeit des jeweils eingesetzten Auftrags- bzw. Druckverfahrens eingestellt wird und der Anteil des Elektrodenmaterialpulvers zwischen 10 Massen-% und 70 Massen-% in der Paste beträgt.

Das dem Elektrodenmaterial zur Erhöhung der Haftung der Elektroden auf dem Substrat zugesetzte anorganische Pulver kann vorzugsweise auch als eine von dem Substratmaterial abweichende Glaskeramik ausgeführt sein, die aus einer Glasmatrix und aus in dieser eingelagerten inerten Bestandteilen, wie Aluminiumoxiden oder anderen geeigneten Materialien, gebildet ist. Bei der Auswahl des anorganischen Materials ist eine Abstimmung hinsichtlich einer guten Haftung der Elektroden auf dem Substrat und einer unerwünschten Verringerung der Leitfähigkeit der Elektroden durch das anorganische Material durchzuführen. Die isolierende Wirkung des anorganischen Materials stellt sich nach dem Einbrennen ein, da es nicht nur einen Stoffschluß mit dem Substrat ausbildet, sondern die Funktionspartikel der Elektroden wenigstens teilweise als isolierende Schicht umgibt.

Nach dem Auftragen der Elektrodenpasten wird das Substrat 3 mit den darauf angeordneten Elektroden 4 und 5 in einem Brennprozeß getrocknet und gebrannt, wobei das zuvor mit dem Pulvergemisch vermengte Vehikel aus den Elektroden verdampft. Während des Brennprozesses wird zwischen den Elektroden 4 und 5 und dem Substrat 3 eine sehr feste Verbindung hergestellt, so daß die Elektroden 4 und 5 selbst bei hohen mechanischen Belastungen bzw. Schwingbelastungen auf dem Substrat 3 sicher verbleiben.

Besteht das Substrat 3 aus einer niedrigsinternden Glaskeramik und sind die Elektroden 4 und 5 aus Iridiumoxid- und/oder Silberpasten hergestellt, wird der Brennprozeß mit Prozeßtemperaturen kleiner 1000°C, vorzugsweise bei 950°C, durchgeführt.

Das Brennverfahren sowie die gewählte Materialpaarung aus Substrat und den Elektroden sind jeweils derart ausgeführt, daß ein während des Brennprozesses auftretender Schwund des Substrates und der Elektroden nur in engen Bereichen voneinander abweicht. Damit werden ein Abplatzen der Elektroden von dem Substrat sowie Risse in den Elektroden, die bei unterschiedlichem Schwundverhalten der Elektroden und des Substrats auftreten, vorteilhafterweise vermieden.

Die in Figur 1 dargestellte Elektrode 4 ist vorliegend aus Silber und die Elektrode 5 ist aus Iridiumdioxid gebildet. Hierzu alternative und ebenfalls für die Herstellung der Elektroden 4 und 5 geeignete Elektrodenmaterialien sind Metalle wie Platin oder Rhodium, sowie Oxide, wie Iridiumoxid, Rutheniumdioxid, Chromtrioxid oder Eisentrioxid. Die verwendeten Elektrodenmaterialien sind durch eine gewisse elektrische Leitfähigkeit gekennzeichnet, so daß über der Meßstrecke nicht zu viel Spannung abfällt bzw. der Innenwiderstand der gesamten Meßzelle kleiner ist als der des Meßmediums.

Die nach dem Einbrennprozeß erhaltene Struktur der potentiometrischen Sensoreinrichtung 1, welche vorliegend aus der Silberelektrode 4, der Iridiumdioxidelektrode 5 und dem aus der Glaskeramik hergestellten Substrat 3 besteht, ist direkt zur Messung verwendbar.

Alternativ hierzu ist es möglich, die Sensoreinrichtung mit einem elektrochemischen Oxidationsverfahren in einem HCl- oder HBr-Bad oder in jedem anderen Halogenid-Ionen-Bad zu behandeln, um die Silberelektrode 4 an deren Oberfläche zu einem Silberhalogenid zu oxidieren und damit zu passivieren. Dadurch werden unerwünschte Reaktionen der reinen Silberelektrode mit der Flüssigkeit, von welcher der pH-Wert gemessen werden soll, vermieden bzw. stark reduziert. Ist die Passivierungsschicht der Silberelektrode 4 eine Silberchloridschicht, weist die Silberelektrode 4 im halogenisierten Bereich ein geringes Stoff-Austauschgleichgewicht mit der Umgebung auf, womit eine besonders beständige Elektrode vorliegt.

Die Iridiumdioxidelektrode 5 verändert sich während des Halogenisierungsprozesses der Silberelektrode 4 nicht und liegt weiterhin in unveränderter Form vor. Die derart ausgeführte potentiometrische Sensoreinrichtung 1 zur pH-Wert-Messung ist durch ein nahezu ideales Nernst'sches Verhalten (Delta_E = -0,059 V * pH) gekennzeichnet, womit auch ohne Signalkonvertierung sehr genaue Messungen durchführbar sind.

Da die erfindungsgemäße Sensoreinrichtung in nicht wässrigen Medien eingesetzt wird, wird die Elektrodenstruktur, d. h. die in Figur 2 dargestellte und gestrichelt umrahmte interdigitale Kammstruktur 6, welche in das nicht wässrige Medium eintaucht, mit einer wasserhaltigen Schicht bzw. einem sogenannten Coating überzogen. Für das Coating sind im Polyamide, wie PA 4, PA 4.4, PA 6, PA 6.6, PA 12, Polyimid, Polyacrylat, Polyethylenglykol oder auch Zellulosen und Zellulosederivate verwendbar. Prinzipiell wird für die auf der Elektrodenstruktur aufgetragene wasserhaltige Schicht ein Polymer-Gel verwendet, das mit Wasser versehen ist.

Wird die Sensoreinrichtung 1 mit der Interdigitalstruktur 6 und der darauf angeordneten wasserhaltigen Schicht mit einem nicht wässrigen Medium beaufschlagt, stellt sich an der Grenze zwischen der wasserhaltigen Schicht und dem nicht wässrigen Medium ein Gleichgewicht zwischen der Wasserstoffionenkonzentration im Coating und der des nicht wässrigen Mediums ein, wodurch eine indirekte Messung erfolgen kann. Damit ist die erfindungsgemäße Sensoreinrichtung insbesondere auch für eine Messung des pH-Werts im Motoröl geeignet, wobei bei Motorölen eine sogenannte Total Base Number (TBN) oder eine sogenannte Total Acid Number (TAN) jeweils technische Synonyme für den pH-Wert darstellen.

Da es sich bei dem zu überwachenden Meßmedium insbesondere um Motoröl handelt, werden die Messungen bei unterschiedlichen Betriebszuständen durchgeführt. Das bedeutet, daß die Messungen bei unterschiedlichen Temperaturen des Motoröls durchgeführt werden. Da insbesondere ein Wassergehalt des Motoröls temperaturabhängig ist, variieren die über die erfindungsgemäße Sensoreinrichtung ermittelten Meßwerte bei verschiedenen Temperaturen des Motorsöls. Dies führt unter Umstanden dazu, daß dem Motoröl über eine Auswertung der Meßwerte unterschiedliche Motorölqualitäten zugewiesen werden, obwohl faktisch keine Veränderung der Qualität des Motoröles stattgefunden hat. Um dies zu umgehen, kann es vorgesehen sein, daß die über die erfindungsgemäße Sensoreinrichtung ermittelten Meßwerte in Abhängigkeit eines aktuellen Betriebszustandes korrigiert werden, um Meßwerte verschiedener Betriebszustände miteinander vergleichen zu können. Mit einer solchen Vorgehensweise stehen über den gesamten Betriebsbereich einer Brennkraftmaschine eines Kraftfahrzeuges miteinander vergleichbare Meßwerte der erfindungsgemäßen Sensoreinrichtungen zur Verfügung.

Eine derartige Kompensation der veränderlichen Betriebsparameter, wie des über den Betriebsbereich einer Brennkraftmaschine veränderlichen Wassergehaltes des Motoröles und der sich ändernden Betriebstemperatur des Motoröls, kann mittels einer entsprechenden Auswerteschaltung durchgeführt werden, die vorzugsweise direkt auf dem Substrat der Sensoreinrichtung angeordnet ist.

Dabei kann es vorgesehen sein, daß der Wassergehalt des Meßmediums entweder direkt über eine Leitfähigkeitsbestimmung sensorisch ermittelt wird oder in Abhängigkeit von Meßwerten einer die aktuelle Temperatur des Motoröles bestimmenden Temperaturmeßeinrichtung über in entsprechenden Speichermedien der Sensoreinrichtung abgelegten Kennlinien ermittelt wird.

Das Coating des Sensoreinrichtung 1 ist vorliegend aus einem ölbeständigen und gleichzeitig wasserhaltigen Polymer hergestellt, so daß die Eigenschaften der wasserhaltigen Schicht durch den Kontakt mit dem Motoröl, der in etwa der Lebensdauer der Brennkraftmaschine entspricht, nicht verändert werden.

Die wasserhaltige Schicht kann auf einer als reine Silberelektrode ausgeführten Elektrode sowie auf einer Passivierungsschicht, beispielsweise auf einer halogenisierten Oberfläche der Silberelektrode aufgebracht sein, wobei die wasserhaltige Schicht auf einer reinen Silberelektrode ebenfalls eine Passivierungsschicht darstellt.

Die Elektroden, die mittels Dickschichttechnik auf dem Substrat aufgetragen werden, weisen im Bereich der interdigitalen Kammstrukturen 6 der Elektroden 4 und 5 Abstände D zwischen 0,1 µm und 1000 µm auf, wobei Abstände zwischen 160 µm und 200 µm - vorzugsweise von 180 µm - im Motoröl besonders gute Ergebnisse bei der pH-Wert-Messung ergeben.

Die beiden Elektroden 4 und 5 sind mit einem ebenfalls auf dem Substrat 3 angeordneten Siliziumchip 8 der Auswerteschaltung 2 verbunden, der mit weiteren auf dem Substrat 3 angeordneten Peripherie-Bauelementen der Auswerteschaltung, wie Widerständen, Kondensatoren und dergleichen, in Kontakt steht. Zusätzlich ist die Sensoreinrichtung mit sogenannten Terminals 10 zur Signal-Weitergabe an ein entsprechendes Steuergerät eines Kraftfahrzeuges, beispielsweise über einen CAN-Bus, ausgeführt.

Im Betrieb der erfindungsgemäßen Sensoreinrichtung generieren die beiden Elektroden 4 und 5 potentiometrische Signale, welche vorliegend jeweils zu einer als ein anwendungsspezifischer integrierter Schaltkreis (ASIC) ausgebildeten Auswerteschaltung geleitet werden, die mit einem MOSFET-Eingang ausgeführt ist und einen Innenwiderstand von mindestens 10¹² Ohm aufweist.

Einer derartige Ausgestaltung der erfindungsgemäßen Sensoreinrichtung ist vorteilhafterweise derart ausführbar, daß der sensierende Bereich der Sensoreinrichtung, d. h. die interdigitalen Kammstrukturen der Elektroden 4 und 5, sehr nahe an der Auswerteschaltung angeordnet sind. Damit wird erreicht, daß ein Spannungsabfall in den Leiterbahnen zwischen den interdigitalen Elektrodenstrukturen und der Auswerteschaltung gegen Null geht.

Die vorliegend zur Herstellung des Substrats 3 verwendete Glaskeramik ist mit in eine Glasmatrix eingebetteten keramischen Partikeln ausgeführt. Die eingebetteten keramischen Partikel erhöhen einerseits die Wärmeleitfähigkeit des Substrats und führen andererseits dazu, daß das Substrat mit einem hohen E-Modul sowie mit einer ausreichenden Härte ausgeführt ist. Die die keramischen Partikel umgebende Glasmatrix der Glaskeramik verleiht dem Substrat eine sehr gute elektrische Isolation und eine sehr niedrige Verdichtungstemperatur.

Zudem ist ein derartiges Substrat während eines Brennprozesses dadurch gekennzeichnet, daß sich bei den für diese Substrate üblichen Heißpreßtechniken lediglich die Höhe des Substrates ändert und eine Länge und Breite des Substrates während des Einbrennprozesses nahezu unverändert bleibt.

Mit der erfindungsgemäßen Sensoreinrichtung ist z. B. ein Ölwechsel im Gegensatz zu bisherigen, fest vorgegebenen Ölwechselintervallen in Abhängigkeit eines aktuellen Zustandes des Motoröls durchführbar. Damit kann einer in Abhängigkeit von Betriebseinsatzbedingungen stehenden vorzeitigen oder späteren Ölalterung Rechnung getragen werden, wodurch unnötig vorzeitige Ölwechsel, die eine Umweltbelastung darstellen und auch eine Erhöhung der Betriebskosten zur Folge haben, vorteilhafterweise vermieden werden können.

## Patentansprüche

1. Potentiometrische Sensoreinrichtung (1) zur pH-Wert-Messung mit auf einem Substrat (3) angeordneten und mittels Dickschichttechnik aufgebrachten Elektroden (4, 5), wobei die Elektroden (4, 5) auf dem Substrat (3) eine Interdigitalstruktur (6) ausbilden, **dadurch gekennzeichnet, dass** auf den Elektroden (4, 5) eine wasserhaltige Schicht vorgesehen ist, die aus Polyamid, vorzugsweise PA 4, PA 4.4, PA 6, PA 6.6 oder PA 12, Polyimid, Polyacrylat, Polyethylenglykol, Zellulose oder einem Zellulosederivat besteht.

2. Potentiometrische Sensoreinrichtung nach Anspruch 1, **dadurch gekennzeichnet, dass** die Elektroden (4, 5) im Bereich der Interdigitalstruktur (6) einen Abstand zwischen 0,1 µm und 1000 µm aufweisen.

3. Potentiometrische Sensoreinrichtung nach Anspruch 2, **dadurch gekennzeichnet, dass** der Abstand 160 µm bis 200 µm, vorzugsweise 180 µm, beträgt.

4. Potentiometrische Sensoreinrichtung nach einem der Ansprüche 1 bis 3, **dadurch gekennzeichnet, dass** das Substrat (3) aus einer Folie aus Glaskeramik hergestellt ist, die eine niedrige elektrische Leitfähigkeit und eine hohe mechanische Stabilität aufweist.

5. Potentiometrische Sensoreinrichtung nach einem der Ansprüche 1 bis 4, **dadurch gekennzeichnet, dass** das Substrat (3) aus einer niedrigsinternden, vorzugsweise bei unter 1000 °C aushärtenden Glaskeramik gebildet ist.

6. Potentiometrische Sensoreinrichtung nach einem der Ansprüche 1 bis 5, **dadurch gekennzeichnet, dass** die Elektroden (4, 5) aus Metallen und/oder Metalloxiden, vorzugsweise aus Silber und Iridiumdioxid, bestehen.

7. Potentiometrische Sensoreinrichtung nach einem der Ansprüche 1 bis 6, **dadurch gekennzeichnet, dass** eine aus Silber bestehende Elektrode (4) wenigstens im Bereich der Interdigitalstruktur (6) an ihrer Oberfläche mit einer Silberhalogenidschicht ausgeführt ist.

8. Potentiometrische Sensoreinrichtung nach einem der Ansprüche 1 bis 7, **dadurch gekennzeichnet, dass** die Elektroden (4, 5) in Form von Pasten auf dem Substrat (3) auftragbar sind, wobei die Pasten zur Haftverbesserung zwischen den Elektroden (4, 5) und dem Substrat (3) mit einem anorganischen Material mit einem Anteil von 0,2 Massen-% bis 20 Massen-%, vorzugsweise 10 Massen-% bis 15 Massen-%, versehen sind.

9. Potentiometrische Sensoreinrichtung nach Anspruch 8, **dadurch gekennzeichnet, dass** das anorganische Material dem Substratmaterial entspricht.

10. Potentiometrische Sensoreinrichtung nach Anspruch 8 oder 9, **dadurch gekennzeichnet, dass** die Pasten aus einem Pulvergemisch aus Elektrodenmaterial und anorganischem Material sowie einem Vehikel gebildet sind, wobei ein Anteil des Pulvergemisches zwischen 10 Massen-% bis 70 Massen-% in der Paste entspricht.

## Claims

1. Potentiometric sensor device (1) for pH value measurement comprising electrodes (4, 5) arranged on a substrate (3) and applied by means of thick-film technology, the electrodes (4, 5) on the substrate (3) forming an interdigital structure (6), **characterized in that** a water-containing layer consisting of polyamide, preferably PA 4, PA 4.4, PA 6, PA 6.6 or PA 12, polyimide, polyacrylate, polyethylene glycol, cellulose or a cellulose derivative is provided on the electrodes (4, 5).

2. Potentiometric sensor device according to Claim 1, **characterized in that** the electrodes (4, 5) in the region of the interdigital structure (6) have a spacing of between 0.1 µm and 1000 µm.

3. Potentiometric sensor device according to Claim 2, **characterized in that** the spacing is 160 µm to 200 µm, preferably 180 µm.

4. Potentiometric sensor device according to any of Claims 1 to 3, **characterized in that** the substrate (3) is produced from a film composed of glass ceramic, said film having a low electrical conductivity and a high mechanical stability.

5. Potentiometric sensor device according to any of Claims 1 to 4, **characterized in that** the substrate (3) is formed from a low-sintering glass ceramic, which preferably cures at below 1000°C.

6. Potentiometric sensor device according to any of Claims 1 to 5, **characterized in that** the electrodes (4, 5) consist of metals and/or metal oxides, preferably of silver and iridium dioxide.

7. Potentiometric sensor device according to any of Claims 1 to 6, **characterized in that** an electrode (4) consisting of silver, at least in the region of the interdigital structure (6), is embodied with a silver halide layer at its surface.

8. Potentiometric sensor device according to any of Claims 1 to 7, **characterized in that** the electrodes (4, 5) can be applied in the form of pastes on the substrate (3), the pastes being provided with an inorganic material with a proportion of 0.2% by mass to 20% by mass, preferably 10% by mass to 15% by mass, in order to improve adhesion between the electrodes (4, 5) and the substrate (3).

9. Potentiometric sensor device according to Claim 8, **characterized in that** the inorganic material corresponds to the substrate material.

10. Potentiometric sensor device according to Claim 8 or 9, **characterized in that** the pastes are formed from a powder mixture composed of electrode material and inorganic material and also a vehicle, a proportion of the powder mixture corresponding to between 10% by mass and 70% by mass in the paste.

## Revendications

1. Capteur potentiométrique (1) pour la mesure d'un pH à l'aide d'électrodes (4, 5) disposées sur un substrat (3) et mises en place au moyen d'une technique à couche épaisse, les électrodes (4, 5) formant sur le substrat (3) une structure interdigitale (6), **caractérisé en ce qu'**une couche aqueuse est prévue sur les électrodes (4, 5), ladite couche étant composée de polyamide, de préférence de PA 4, PA 4.4, PA 6, PA 6.6 ou PA 12, de polymide, de polyacrylate, de polyéthylène glycol, de cellulose ou d'un dérivé cellulosique.

2. Capteur potentiométrique selon la revendication 1, **caractérisé en ce que** les électrodes (4, 5) présentent un écartement compris entre 0,1 µm et 1000 µm dans la zone de la structure interdigitale (6).

3. Capteur potentiométrique selon la revendication 2, **caractérisé en ce que** l'écartement est de 160 µm à 200 µm, de préférence de 180 µm.

4. Capteur potentiométrique selon l'une quelconque des revendications 1 à 3, **caractérisé en ce que** le substrat (3) est fabriqué à partir d'un film en vitrocéramique qui présente une faible conductivité électrique et une grande stabilité mécanique.

5. Capteur potentiométrique selon l'une quelconque des revendications 1 à 4, **caractérisé en ce que** le substrat (3) se compose d'une vitrocéramique à faible vitrification durcissant de préférence à une température inférieure à 1000 °C.

6. Capteur potentiométrique selon l'une quelconque des revendications 1 à 5, **caractérisé en ce que** les électrodes (4, 5) sont en métal et/ou en oxyde de métal, de préférence en argent et en dioxyde d'iridium.

7. Capteur potentiométrique selon l'une quelconque des revendications 1 à 6, **caractérisé en ce qu'**une électrode (4) en argent est réalisée avec une couche d'halogénure d'argent, et ce au moins dans la zone de la structure interdigitale (6), au niveau de sa surface.

8. Capteur potentiométrique selon l'une quelconque des revendications 1 à 7, **caractérisé en ce que** les électrodes (4, 5) peuvent être appliquées sous forme de pâtes sur le substrat (3), les pâtes étant pourvues d'un matériau présentant une teneur de 0,2 % en masse à 20 % en masse, de préférence 10 % en masse à 15 % en masse, pour améliorer l'adhérence entre les électrodes (4, 5) et le substrat (3).

9. Capteur potentiométrique selon la revendication 8, **caractérisé en ce que** la matière anorganique correspond à la matière du substrat.

10. Capteur potentiométrique selon la revendication 8 ou 9, **caractérisé en ce que** les pâtes sont formées d'un mélange de poudre composé de matière d'électrode et de matière anorganique ainsi que d'un excipient, la partie du mélange de poudre représentant entre 10 % en masse à 70 % en masse de la pâte.
